# EUROPEAN PATENT SPECIFICATION

(11) **EP 3 878 448 B1**
(45) Date of publication and mention of the grant of the patent: **07.01.2026**
(21) Application number: 19882109.2
(22) Date of filing: 01.11.2019
(51) Int. Cl.: A61K 31/415, A61K 31/635, A61K 31/27, A61P 29/00, A61P 21/02, A61K 9/00, A61K 45/06

(54) **ORAL PHARMACEUTICAL COMPOSITION COMPRISING CELECOXIB AND METHOCARBAMOL AND ITS USE FOR TREATING PAIN, INFLAMMATION AND MUSCULAR CONTRACTION**
ORALE PHARMAZEUTISCHE ZUSAMMENSETZUNG, UMFASSEND CELECOXIB UND METHOCARBAMOL UND IHRE VERWENDUNG ZUR BEHANDLUNG VON SCHMERZEN, ENTZÜNDUNGEN UND MUSKELKONTRAKTION
COMPOSITION PHARMACEUTIQUE ORALE COMPRENANT DU CÉLÉCOXIB ET DU MÉTHOCARBAMOL ET SON UTILISATION POUR LE TRAITEMENT DE LA DOULEUR, DE L'INFLAMMATION ET DES CONTRACTURES MUSCULAIRES

(30) Priority: 05.11.2018 MX 2018013458
(43) Date of publication of application: 15.09.2021
(73) Proprietor: Amézcua Amézcua, Federico, Guadalajara, Jalisco, 44898 (MX); Amézcua Amézcua, Carlos, Guadalajara, Jalisco, 44898 (MX)
(72) Inventor: GARCÍA ARMENTA, Patricia del Carmen, Zapopan, Jalisco, 45019 (MX); CHÁVEZ GARCÍA, José Alonso, Zapopan, Jalisco, 45138 (MX)
(74) Representative: Ungria López, Javier
(86) International application number: PCT/MX2019/000117
(87) International publication number: WO 2020/096441

(56) References cited:
- WO-A1-01/91750
- WO-A1-2007/037666
- MX-A- 2016 017 000
- US-A1- 2004 204 413
- US-A1- 2007 213 324
- TISDALE S A JR ET AL: "A CONTROLLED STUDY OF METHOCARBAMOL (ROBAXIN) IN ACUTE PAINFUL MUSCULOSKELETAL CONDITIONS", CURRENT THERAPEUTIC RESEARCH, EXCERPTA MEDICA, TRENTON, NJ, US, vol. 17, no. 6, 1 June 1975 (1975-06-01), pages 525 - 530, XP008079219, ISSN: 0011-393X

## Description

### FIELD OF THE INVENTION

The present invention is related to the technical field of the pharmaceutical industry, and its object is to provide a pharmaceutical composition comprising the synergic pharmaceutical combination of a selective inhibitor of cyclooxygenase 2, constituted by the active ingredient celecoxib or pharmaceutically acceptable salts thereof and a carbamate derived from guaifenesin constituted by the active ingredient methocarbamol or a pharmaceutically acceptable salt thereof, to be administered with pharmaceutically acceptable excipients, adjuvants or additives, formulated in a single solid or liquid dosing unit to be administered orally, for pharmacological uses in the treatment of pain, inflammation and muscular contraction.

The combination of the aforementioned active principles in the specific amount given exhibits synergetic improved properties, produces a triple effect: analgesic, anti-inflammatory and muscle relaxant when said active principles are administered jointly, in a single dosing unit, instead of being administered separately; and provides benefits, such as a lower administered dose, a greater therapeutic effect, less side effects and a synergic effect, and finally it is better tolerated.

### BACKGROUND OF THE INVENTION

There is a large group of muscle and aponeurosis (reinforcement of the tendon and muscle unions) pain forms, characterized by pain points or trigger points in one or more muscles or unions in different areas of the body. These pain forms are usually accompanied by muscle contraction, impairment of column, neck, waist and limb movement, and in some cases anatomic alterations of muscles and joints. Physicians define them as musculoskeletal or myofascial pain, and said pain points in muscles are the most frequent ailments of patients when seeking help from pain specialists, who can detect these pain points by simple palpation.

For its physiopathology, the most widely accepted theory is the "energy crisis model", according to which an excessive acetylcholine release in motor end-plates causes a continuous contraction of certain sarcomeres, which in turn conditions a greater energy demand in muscles, a local ischemia causing: a) an increasing sensitization of primary afferent nociceptors in the affected area, b) an increasing presence of proinflammatory neurotransmitters and allogenic substances affecting transduction, causing peripheric sensitization. If this phenomenon persists, it can also affect transmission and modulation in the spinal cord's dorsal horn. Etiologic agents involving microtrauma or large traumas alter normal or weakened muscles, releasing free calcium inside muscles along the sarcoplasmic reticulum. Adenosine triphosphate (ATP) stimulates the actin and myosin interaction with local metabolic and contractile activity, thus increasing the production of noxious degradation elements such as serotonin, histamine, kinins and prostaglandins. Simultaneously, group **III** and group IV muscle nociceptors are sensitized and activated, establishing a neural circuit between nociceptors, the central nervous system and motor units.

These afferent impulses converge with visceral and somatic impulses, which explains the perception of local pain.

This cycle can persist by a protective action of the aching muscle through a distorted muscle posture (antalgic posture). Sustained contractile activity decreases the local blood flow, causing low oxygen levels, ATP reserve depletion and diminished calcium pump activity. Free calcium keeps interacting with ATP, triggering a non-actin contractile activity, resulting in a vicious circle.

This disease affects persons of both sexes, although its incidence is higher in women of 30-55 years of age, sedentary working habits, little physical activity and who occasionally decide to exercise vigorously without properly preparing their muscles.

The most affected muscles, with persistent pain and variable intensity, are mostly located in the head, neck, shoulders, limbs, gluteus and lower back.

The disease is often associated with musculoskeletal diseases, arthritis, nerve lesions and visceral illnesses.

### Classification:

When considering muscle lesions according to their origin, they can be classified in two major groups:
1. Lesions with no traumatic components, in which discomfort or physical impairment such as cramps and contractures are caused by overexercising.
2. Lesions caused by traumatism, whether external (such as muscle contusions) or internal (such as muscle pulls, strains, swellings or total or partial fiber tears).

### Muscle contractures:

A muscle contracture is defined as an increase of muscular tone in a specific area. The contracture of a muscle or several muscles, if left unattended for an extended period with no relaxation, causes pain. The contracture is the result of an irritating cause, and most of these cases occurring in shoulders, and in the upper, middle and lower back are due to incorrect bodily postures. Forward bending postures, if maintained for an extended period, exhausts the muscles that bear the load of these stances. Because of this exhaustion muscles lose their ability to relax, leading to more exhaustion and thus creating a hard-to-break vicious circle.

### Swellings:

Swellings are caused by excessive pressure on muscle fibers exerted by sudden movements (jerks) on the verge of tearing the fibers (fibrillar pre-rupture).

### Fibrillar tears or ruptures:

Most commonly, these are partial ruptures with few fibers involved. In cases of major injuries massage is absolutely contraindicated. It is essential to accurately assess the lesions since if there are large gaps between the muscle's injured ends, conservative treatments are unsuccessful and surgery can be required, since in these cases muscle healing cannot be taken for granted and major sequels may appear.

Other cases are old muscle injuries with incompletely healed minor or major tears, which are always weak areas where lesions can reappear, or are more exposed to new injuries.

### Acute pain syndrome:

This condition is a continual or intermittent painful spasm in the shoulder or neck muscles, which forces the head to rotate or tilt forward, backwards or sidewards.

Pain syndromes affect one in every 10,000 persons and are 10 times more frequent in women than in men. Although this disorder is not age-related, its incidence is greater between 30 and 60 years old.

### Bursitis:

Bursitis is the inflammation with pain of a bursa (synovial fluid-filled sacs which facilitate the normal movement of some joints and muscles by reducing friction).

Bursae are located in friction points, especially in tendons or muscles directly superposed on bones. Although bursae contain little fluid, lesions can inflame them and overfill them with fluid.

Bursitis can be caused by chronic excessive use of a joint, or as a result of injuries, gout, pseudogout, rheumatoid arthritis or infections, although the cause is often unknown. Shoulders are more prone to bursitis, although bursae inflammations in elbows, hips, pelvis, knees, toes and ankles are also frequent.

### Tendinitis and tenosynovitis

Tendinitis is the inflammation of tendons; tenosynovitis is tendinitis plus inflammation of the protective sheath surrounding a tendon.

Tendons, some of which are lined by a protective sheath, are fibrous cords of resilient tissue connecting muscles to bones. The protective sheaths of the tendons surround some tendons.

Most tendinitis cases appear in middle- or old-aged adults, since tendons are more prone to lesions with age. However, it also can be observed in intensely exercising young persons and individuals performing repetitive tasks.

Certain tendons, especially in the hands, are particularly prone to inflammation. The inflammation of tendons in the hand's first dorsal tunnel, which control the movements of the thumb, is known as De Quervain syndrome or stenosing tenosynovitis. The tendons controlling the other fingers can also be trapped by this inflammation, causing a cracking sensation (trigger finger).

Biceps tendinitis, in the forearm proximal part, causes pain whenever the elbow is flexed or when rotating the forearm. When affecting the long portion of the biceps in the shoulder's anterior side, it impairs its mobility, particularly when flexed.

Inflammation of the Achilles and toe extensor tendons is frequent.

Delayed onset muscle soreness (DOMS) is regularly caused whenever an individual engages in non-habitual exercising, regardless of being a sportsperson or not. Armstrong (1984) defines DOMS as a discomfort or muscle pain occurring after unaccustomed exercising. It is more common in athletes starting their training seasons when intense training is reintroduced after a period of relative inactivity. At the beginnings of the last century, Hough (1900) noted two types of muscle pain after engaging in physical activity. The first was a sharp pain appearing during or immediately after intense physical activities, and its cause could be attributable to waste products accumulation.

The second type, occurring at least eight hours after exercising, with a peak in pain intensity 24 to 48 hours thereafter, could be attributable to muscle fibers ruptures.

Undoubtedly DOMS exerts negative effects on the performance of sportspersons and individuals who exercise occasionally, and for this reason researchers have been seeking for strategies to minimize it, whether preventive (administering them before physical activities) or therapeutic (post-physical activities administration). Many studies have analyzed the nutraceutical effects of blueberries, caffein, sea oil complex, eicosapentaenoic acid, pomegranate juice and branched-chain amino acids. Other studies have focused on the effects of physical therapies such as hydrotherapy, cryotherapy, humid and dry heat, vibrations and electric microcurrents. However, massage and nonsteroidal anti-inflammatory drugs (NSAIDS) are the most cited therapies in studies focused on countering DOMS.

The theory that better explains DOMS is muscle injury caused by several mechanisms, described as: tensile stresses exerted during muscle fibers contraction and more so during eccentric activities, when a muscle elongates under stress, causing rupture of structural proteins in the fibers, particularly those dividing myofibrils in sections known as sarcomeres. After a few hours there is a significant increase in neutrophils circulating at the injury site; 48 hours later there is a peak in the monocyte/macrophage relation, which significantly increases the production and release of prostaglandins (mediators related to pain and inflammatory processes), sensitizing group III and IV nerve terminals located in muscles, joints, ligaments and tendons which cause thermal and pain sensations. The build-up of histamines, potassium and cytokines of active phagocytes, along with cellular necrosis, high pressure of intercellular fluids in tissues (oedema), and an increase in body temperature, activate nociceptors in muscle fibers and tendons.

Besides the evidence of effects on the process of pain, inflammation and muscle contractures, the use of NSAIDS could have an even greater potential.

Presently are available several strategies including pharmacologic treatments of pain, inflammation and muscle pain. The celecoxib-methocarbamol combination has potential therapeutic usefulness for patients suffering from these complications.

Celecoxib is the compound 4-[5-(4-methylphenyl)-3-(trifluoromethyl)-1*H-*pyrazol-1-yl]benzenesulfonamide, represented by formula (I)

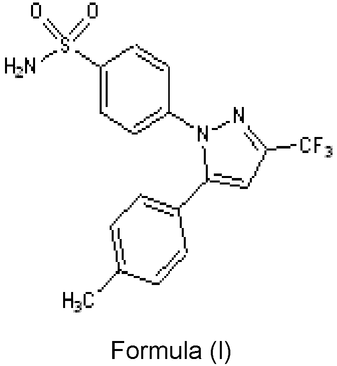

Described for the first time in US Patent 5,466,823 for the treatment of inflammation and associated disorders such as arthritis.

Celecoxib is administered only orally, food does not hinder it absorption, and its peak plasma concentration is reached within 3 hours. The approved daily doses are 100-400 mg, and is a cyclooxygenase 2 (COX-2) selective inhibitor. It has a plasma protein binding of 97% and is extensively metabolized in the liver by isoenzyme CYP2C9 of cytochrome P-450. It is indicated for the symptomatic relief of arthrosis and rheumatoid arthritis. Its effectiveness has been assessed in several clinical trials, including many comparisons with other NSAIDS such as diclofenac, naproxen and ibuprofen, with an effectiveness superior to that of placebo and similar to these compounds.

Celecoxib has a high selectivity for COX-2 receptors, and unlike other non-selective NSAIDS has no COX-1 inhibiting activity at therapeutic concentrations and does not significantly affects platelet aggregation or bleeding time.

It has been proposed that COX-2 selective inhibitors are more advantageous than classic NSAIDS since they cause less gastrointestinal adverse effects.

A recent meta-analysis reviewing large numbers of patients concluded that celecoxib is safer (less incidence of ulcers and complications) than classic NSAIDS. The results of this and other meta-analysis have been reasserted in daily practice; several observational studies based on cohorts or cases and controls have shown that COXIBSs are less associated with hemorrhages than conventional NSAIDS.

This assertion was confirmed by the CLASS trial (Celecoxib Long-term Arthritis Study), which included approximately 8,000 arthrosis and rheumatoid arthritis (RA) patients, of whom 20% were taking low doses of acetylsalicylic acid (ASA) as cardiovascular prophylaxis. GI toxicity was assessed after 6 months of celecoxib treatment (400 mg b.i.d.) compared to ibuprofen (800 mg t.i.d.) and diclofenac (75 mg b.i.d.). The study primary endpoint, incidence of ulcer complications (perforation, obstruction and bleeding), was not significantly different. However, in patients without concomitant ASA celecoxib was associated with a lower GI bleeding incidence. According to its FDA prescribing information, celecoxib has an estimated effective half-life of 8-12 hours.

On the other hand, methocarbamol is the compound 3-(2-methoxyphenoxy)-1,2-propanediol 1-carbamate, represented by formula (II),

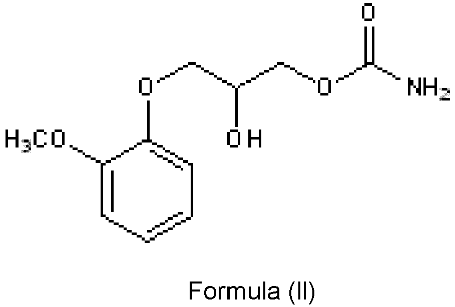

Described for the first time in US Patent US 2,770,649 to be used as a muscle relaxant.

Methocarbamol is a centrally acting muscle relaxant with a not fully understood mechanism of action, although it is believed that it could be due to a CNS depressant effect, reducing the transmission of impulses from the spinal cord to skeletal muscles.

It does not act directly on the striated muscle contractile mechanism, neuromuscular junctions or axons. It is used as adjuvant therapy in the symptomatic treatment of painful muscular spasms associated with musculoskeletal conditions.

Since its introduction to clinical practice, good results were reported as muscle relaxant for herniated lumbar and cervical discs, lumbosacral sprains and contractures.

When administered by oral route it reaches active levels within 30 minutes, with a peak action in humans within 2 hours. Methocarbamol is metabolized by dealkylation and hydroxylation, and the resulting two metabolites are primarily glucuronide and sulfate conjugates.

In the state of the art, US Patent 9,962,336 discloses a stable extended release reconstituted powder for suspension composition comprising multiple coated cores of an active ingredient selected from celecoxib and methocarbamol, wherein upon reconstitution with a suspension base, the composition ensures substantially similar in-vitro dissolution release profile of the active ingredient upon storage of the composition upon reconstitution for at least seven days; wherein the active ingredient is not bound to an ion-exchange matrix; and wherein the suspension base used for reconstitution of the composition is characterized by having the features of: (i) a viscosity in a range of about 500 cps to about 15,000 cps, and (ii) an osmolality of at least 1 osmol/kg of the suspension base; wherein the composition upon reconstitution is characterized by having an osmolality ratio of at least about 1, the osmolality ratio being the ratio of the osmolality of the external phase to the osmolality of the internal phase, the external phase being the suspension base without multiple coated cores of the active ingredient and the internal phase being the coated cores of the active ingredient; wherein the osmolality of the internal phase is the osmolality of a solution which prevents significant leaching of the active ingredient from the coated cores into the solution: wherein the coated cores consist of a core of an active ingredient and a coating layer over said core comprising one or more release-controlling agents and average diameter of the coated cores ranges from about 150 µm to about 500 µm, and wherein the composition is homogeneous and the active ingredient is layered onto an inert particle to form the core. US Patent 9,770,514 discloses an extended release dosage form, comprising: at least one therapeutic agent; at least one substrate; at least one thermoplastic polymer, and at least one pharmaceutical additive, wherein the at least one therapeutic agent and the at least one substrate form a therapeutic agent-substrate complex, wherein the complex is prepared using a reactive extrusion process that results in the formation of complex particles, the at least one thermoplastic polymer and the at least one pharmaceutical additive form a thermo-formable matrix, and the therapeutic agent-substrate complex is embedded in the thermo-formable matrix, wherein the complex is embedded in the matrix using a hot melt extrusion process; wherein the pharmaceutically active agents can be selected from celecoxib and methocarbamol, and the process for preparing said process is also disclosed. US Patent 8,029,822 discloses a rupturing controlled release device comprising: a) a core comprising an active agent and, optionally, at least one excipient, and b) a wall enclosing the core and having a weakened section and a preformed passageway there through, wherein the wall ruptures at the weakened section during use due to an increase of internal osmotic pressure of the core during use of the device to form a second passageway by breakage of the wall at a location spaced away from the preformed passageway such that active agent is released over an extended period of time from both passageways, wherein the active agents can be selected from celecoxib or methocarbamol. EP Patent 1362585 discloses a pharmaceutical composition comprising: a) a COX-II inhibitor; b) a muscle relaxant; and at least one pharmaceutical excipient, wherein the weight ratio of COX-II inhibitor to muscle relaxant varies from 12.5:2.2 to 50:8, wherein the COX-II inhibitor is selected from celecoxib and the muscle relaxant is selected from methocarbamol for the treatment of pain.

Some documents that can be mentioned, related to compositions and treatments for alleviating pain and inflammation derived from tendinitis, bursitis, osteoarthritis or the like, are: WO2007037666 A1, US2004204413 A1, US2007213324 A1, MX2016017000 A, WO2001/91750 and Tisdales A, JR et al (1975). Current Therapeutic Research.

The present invention is characterized by providing a specific synergic combination comprising celecoxib or pharmaceutically acceptable salts thereof and methocarbamol or pharmaceutically acceptable salts thereof, not previously reported in the state of the art. This drug combination allows the patient a prompt return to her/his daily activities. The use of this drug combination offers a synergism which allows a reduction in the required doses and a decrease in adverse effects. The aforementioned active ingredients combination has improved properties, and produces a triple effect: analgesic, anti-inflammatory and muscle relaxant.

### OBJECT OF THE INVENTION

Associations of analgesic and adjuvant agents are widely used in therapeutics, and several of these associations are formulated as an attempt to trigger greater effectiveness with lower doses; and consequently, lower doses cause less adverse effects. The invention as in claim 1 offers a new therapeutic option for the treatment and management of pain, inflammation and muscular contraction, that reduces symptoms and improves the patients' quality of life. It also offers the usefulness of a celecoxib and methocarbamol combination, administered with pharmaceutically acceptable excipients or adjuvants, formulated in a single dosing unit to be administered orally in a solid or liquid form. This combination triggers a synergic interaction with a greater therapeutical potency and an earlier onset of action, while reducing adverse events.

The combination of these active ingredients, in the amount given (a concentration of 1000 mg/kg of celecoxib or pharmaceutically acceptable salts thereof, combined with 50 mg/kg of methocarbamol or pharmaceutically acceptable salts thereof), results in greater pharmacological potency, improving therapeutics and offering benefits such as: the administration of the active ingredients at lower concentrations, than if being administered separately, greater effectiveness and therapeutic potency, and significantly reducing the probability of side effects that could occur if the active ingredients were being independently administered compared to being administered jointly. This combination triggers a triple effect: analgesic, anti-inflammatory and muscle relaxant, is better tolerated and has less adverse effects in patients suffering pain, inflammation and muscular contractions.

### BRIEF DESCRIPTION OF THE DRAWINGS

Figure 1 (Comparative results, for illustration purposes only). Temporal course (TC) of antinociceptive effects produced by oral celecoxib in the experimental pain-induced functional impairment model in the rat; a gout arthritis was induced in rats. There is a dose-dependent relation.
Figure 2 (Comparative results, for illustration purposes only). Dose-response curve (DRC) of oral celecoxib plotted as AUC's of TC's of global antinociceptive effects versus administered doses of the drug. The graph points are expressed as mean ± standard error.
Figure 3 (Invention vs. comparative results). TC's of antinociceptive effects produced by oral celecoxib + methocarbamol 50 mg/kg in the experimental pain-induced functional impairment model in the rat, acute administration.
Figure 4 (Invention vs. comparative results). DRC of the celecoxib + methocarbamol 50 mg/Kg combination by oral route, plotted as AUC's of TC's of global antinociceptive effects versus administered doses of the combined drugs. The graph points are expressed as mean ± standard error.
Figure 5 (Invention vs. comparative results). DRC of celecoxib alone and the celecoxib + methocarbamol 50 mg/Kg combination, plotted as AUC's of TC's of global antinociceptive effects versus administered doses of celecoxib alone and combined with methocarbamol 50 mg/Kg. The graph points are expressed as mean ± standard error.
Figure 6 (Invention vs. comparative results). TC's of antinociceptive effects produced by the most efficacious doses: celecoxib 1,000 mg/Kg p.o., the combination celecoxib 1,000 mg/Kg and methocarbamol 50 mg/Kg p.o., and methocarbamol 50 mg/Kg p.o. in the pain-induced functional impairment in the rat experimental model.

### DETAILED DESCRIPTION OF THE INVENTION

Low to high intensity muscular pain, inflammation and contraction are among the most common ailments in the global population. Presently, treatments of muscular pain, inflammation and contraction are simple, with over-the-counter drugs easily available to patients.

The present invention proves with preclinical tests, that particular doses of the novel celecoxib-methocarbamol combination, as disclosed in claim 1, have an unexpected triple synergic therapeutic effect in the treatment of muscular pain, inflammation and contraction. Thus, the main objective of this invention is to develop the pharmaceutical combination of a selective inhibitor of cyclooxygenase 2, such as the active ingredient celecoxib or pharmaceutically acceptable salts thereof, and a carbamate derived from guaifenesin, such as the active ingredient methocarbamol or pharmaceutically acceptable salts thereof, administered with pharmaceutically acceptable additives and/or excipients and/or adjuvants, formulated in a single dosing unit for oral administration, in solid or liquid form, indicated for the treatment and management of muscular pain, inflammation and contractions (see claim 1).

Nowadays, an alternative for increasing the effectiveness of an analgesic treatment while significantly reducing its side effects, is through the combined administration of two or more active agents, such as this synergic pharmaceutical combination intended to be protected by the present disclosure. The celecoxib and methocarbamol combination is designed to control symptoms, prevent complications and restoring full functionality of a mammal, with minimal adverse or side effects caused by the pharmacological treatment.

The present invention intends to offer a new therapeutical option for the treatment and management of muscular pain, inflammation and contractions, capable of reducing symptoms and improving the patients' quality of life. The aforementioned active principles combination results in triple effects: analgesic, anti-inflammatory and muscle relaxant.

A feature of this invention is that its administration can be increased without complications for a certain period of time in order to avoid relapses or musculoskeletal complications.

The present pharmaceutical combination, whether in its base form or its pharmaceutically acceptable salts, is administered by oral route in solid or liquid form.

Nowadays, there is no assessment of the effects that the celecoxib and methocarbamol combination may cause in the treatment of muscular pain, inflammation and contractions. Accordingly, the object of this invention is to preclinically assess the effectiveness in an in vitro rat model of the antinociceptive effects resulting from the oral celecoxib and methocarbamol combination, and to analyze its possible adverse effects.

This combination improves the therapeutics and offers benefits such as: the administration of the active principles in lower concentrations than if administered separately; more effectiveness, greater therapeutic potency and significantly less probabilities of secondary effects if administered in a combination than if administered separately. Moreover, there is a reduction in secondary effects caused by the separate administration of the compounds, since the doses are lower than those commercially available. Accordingly, the behavior of this combination was proved preclinically, and the interaction and synergism of both compounds were determined along with the optimal proportions of the combination, which had a high degree of effectiveness and therapeutic potentiation, delivering a triple analgesic, anti-inflammatory and muscle relaxant effect.

### Experimental model

### Experiment animals and operational description

In order to determine the possible antinociceptive and toxic effects of the combination, male Wistar rats [Crl:(WI)fBR] with a weight of 180-200 g were used. All experimental procedures complied with the recommendations of the Committee for Research and Ethical Issues of the International Association for the Study of Pain (Covino et al., 1980) and the Guidelines on Ethical Standards for Investigations of Experimental Pain in Animals (Zimmermann 1983). The number of experimental animals was kept to a minimum (6 animals per experimental point for antinociceptive effects and 10 animals per experimental point for adverse effects: lethality). The rats were kept in a room with alternating light/darkness cycles. For twelve hours before the antinociception experiments rats fasted but had free access to water. All experiments were performed during the light phase of the light/darkness cycles.

We determined first the antinociceptive effects of each compound, and then we analyzed the effects of the combination, using the pain-induced functional impairment in the rat model ("PIFIR model").

Animals were anesthetized under an extraction bell in a glass desiccator saturated with ether vapor. Gout arthritis was induced by intra-articular injection (i.art.) of 0.05 mL uric acid suspended in mineral oil (30% dilution) in the right main knee joint of the hindlimb.

For the i.art. injection, 1-mL glass syringes with n. 22 of 4 mm-long needles were used. Immediately after, electrodes were fixed between the sole callosities of each hindlimb. Rats were allowed to recover from anesthesia and then placed in a 30-cm diameter stainless steel rotating cylinder. The cylinder was rotated at 4 rpm, forcing the rats to walk 2 minutes every half hour for a total time of 6.5 hours (2.5 hours to cause arthritis and 4 hours to assess antinociceptive effects). The measured variable was duration of contact of each hindlimb with the cylinder.

The contact of electrodes with the cylinder closed a circuit, and the relation between the duration of contact of the hindlimb with uric acid and that of the hindlimb without uric acid was recorded in a computer.

For the preclinical toxicity (lethality) studies, we first determined for each compound (celecoxib alone and methocarbamol alone) the range of oral doses from the maximal non-lethal dose to the minimal dose causing death in 100% of the laboratory animals (rats) that received the treatment. The lethal toxic effects after single administrations were determined in 3 post-treatment timepoints, 24, 48 and 72 hours, in order to determine the corresponding DRC (death). In the assessments made for each drug alone and for both drugs combined, the treatment was given in a single dose whenever possible, although when this was not possible due to high concentrations and solubility limits, several doses were administered, with a time gap of 3 hours between doses. The established doses were always increased in logarithmic units, both for celecoxib and methocarbamol by oral route.

### Experimental protocol

First it was decided to calculate the dose ranges needed to plot the dose-response curves of celecoxib, and of methocarbamol. It was determined in previous studies that methocarbamol 50 mg/kg is an adequate dose for drug combinations in preclinical trials, and for determining whether there are changes in the antinociceptive effects.

Then we determined and analyzed the antinociceptive effects produced by the compounds given alone or in combination in animals with gout arthritis, using the pain-induced functional impairment in the rat experimental model. Determinations were made under 2 conditions: 1) Acute administration in animals with gout arthritis, 2) Chronic administration (7 days of daily administration) and assessment of antinociceptive in animals with induced gout arthritis.

After administering uric acid in the joints to induce gout-type dysfunction, a waiting period of 2.5 hours was initiated to allow the total dysfunction to set in (diagnostic features of gout arthritis were already clear 2.5 hours after administering uric acid). 2.5 hours after the uric acid administration was considered as timepoint "0", and then began the treatment with drugs alone and in combination. The temporal course of each treatment was determined for the next 4 hours. Each treatment had n = 6 rats. For the purposes of this research induced nociception was inevitable, although special care was taken to avoid unnecessary suffering in animals.

Then the possible effects caused by celecoxib alone or the celecoxib + methocarbamol 50 mg/Kg combination were determined in acute treatments. A similar procedure was employed in animals receiving celecoxib 100 mg/kg or celecoxib 100 mg/Kg - methocarbamol 50 mg/Kg for 7 days. Each compound was given to rats as 20 mL/Kg oral doses.

Data were expressed as Percentage Functionality Index (%FI). %FI is the ratio obtained when dividing the duration of contact of the hindlimb with uric acid by duration of contact of the other hindlimb of the same rat, times 100. Temporal courses (TC) were plotted as %FI versus time (h); in these experimental conditions, analgesic or antinociceptive effects were estimated as recovery in %FI. All graphed values in the Figures are expressed as mean ± standard error for 6 animals.

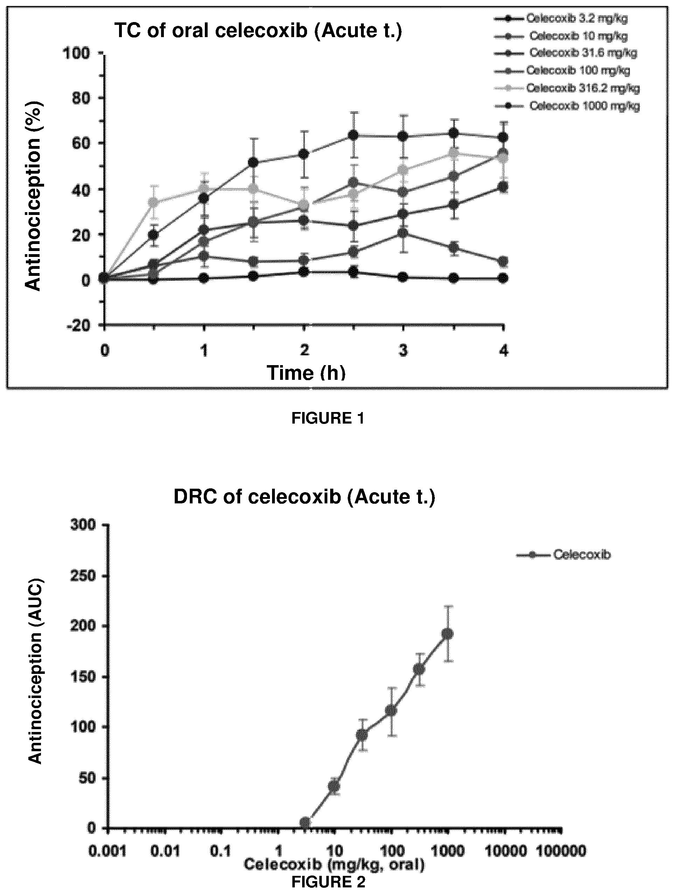

Temporal courses plotted for celecoxib in a single or acute administration over 4 hours in doses with logarithmic increases (3.2 - 1,000 mg/kg): The X-axis is assessment time in hours, while the Y-axis is the antinociceptive effect evaluated as percentage functionality index (%FI). Different doses of celecoxib were individually administered in timepoint "0" (when total or partial hindlimb dysfunction could already be observed in rats). In such experimental conditions dose-dependent antinociceptive effects were evident during the 4-hour observation period (Figure 1).

Area under the curve (AUC) of the temporal course (RTC) of each celecoxib dose was calculated in order to determine its global antinociceptive effects, reflecting both the effects during the 4-hour assessment period and maximal effects produced over time. Celecoxib's dose-response curves (DRC) were constructed with these AUC's and are shown in Figure 2. The X-axis in this Figure is doses expressed as 0.5 units-logarithmic increases, while the Y-axis is global antinociceptive effects (AUC) plotted to construct the DRC. Means and standard errors were graphed, and the complete sigmoid curve can be observed, which is oral celecoxib's DRC. The peak antinociceptive effectiveness obtained was 191.9 ± 27.2 area units (au) (Figure 2).

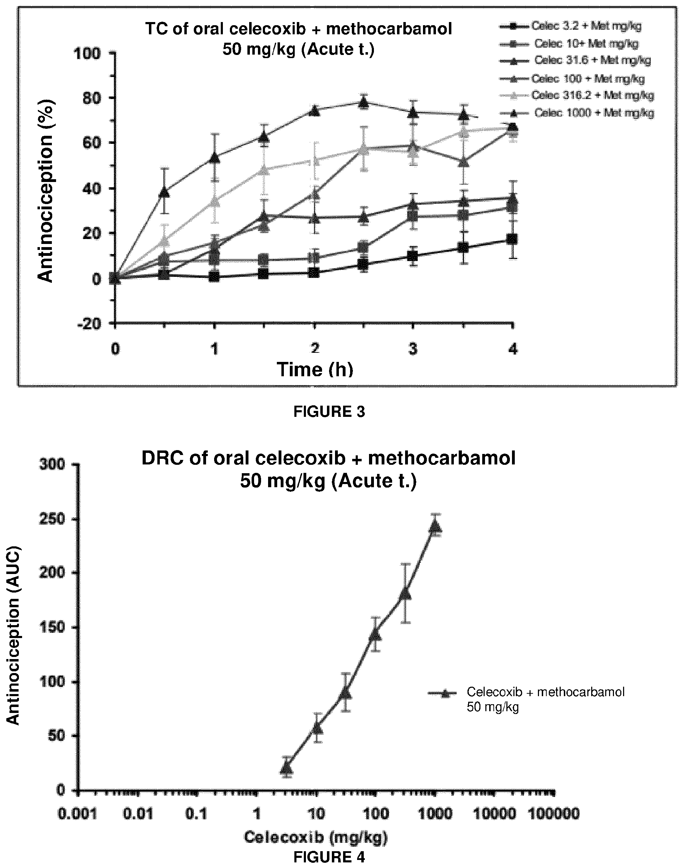

Then the temporal courses of celecoxib simultaneously administered with methocarbamol 50 mg/Kg by oral route in acute treatment were determined. The axes are as described in Figure 1, and it may be noticed that dose-dependent effects were also observed (Figure 3).

Based on the different time courses of celecoxib + methocarbamol 50 mg/Kg, AUC'S were again calculated to obtain a measure of the global antinociceptive effect, and the corresponding DRC was constructed. When graphing the means and standard errors of the global effects in Figure 4, it can be observed that the maximal effectiveness obtained with the celecoxib 1,000 mg/kg - methocarbamol 50 mg/Kg combination was 244.0 ± 9.7 area units (au) (Figure 4).

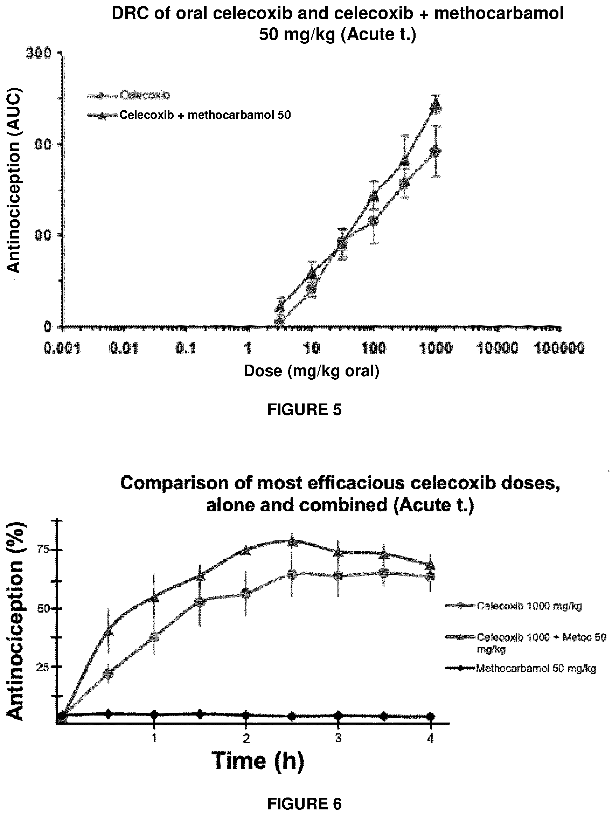

DRCs of celecoxib alone (for illustration purposes only) and of celecoxib + methocarbamol 50 mg/Kg were compared. The line with circles is the DRC of celecoxib alone, and the line with triangles is the DRC of the celecoxib + methocarbamol 50 mg/Kg combination, and it can be noted that the combination's DRC is slightly displaced to the left, and that there is a trend toward a better effectiveness than the effectiveness resulting from the same doses of celecoxib alone. Thus, the association with methocarbamol produced a better antinociceptive effect, and tended to improve effectiveness in comparison with the results of the administration of celecoxib alone. When analyzing ED₅₀'s it was found that the administration of celecoxib alone has an ED₅₀ of 51.1 mg/Kg, while the ED₅₀ of celecoxib + methocarbamol 50 mg/Kg was 70.5 mg/Kg, i.e., somewhat higher, although calculated based on an improved effectiveness (Figure 5).

After comparing DRCs of acute administrations, a comparison of the most noteworthy TCs of acute administration was performed. TCs of the most efficacious doses observed in the DRC, triggered by celecoxib alone and combined with methocarbamol 50 mg/Kg were compared. Methocarbamol 50 mg/Kg alone (shown as a line with triangles, for illustration purposes only) did not produce any antinociceptive effect whatsoever during the assessment period. Again, the line with circles is the TC of celecoxib 1,000 mg/Kg alone, which was the most efficacious dose (when administering celecoxib alone), and the line with triangles is the TC of the celecoxib 1,000 mg/Kg - methocarbamol 50 mg/Kg combination (illustrating the present invention as in claim 1), which was the most efficacious dose of the celecoxib and methocarbamol combination. A tendency toward a faster onset of action can be noted for the combination, which is maintained up to 3 hours after the administration. After this period, the combination's antinociceptive effect diminishes and equals the effects of celecoxib alone, whose antinociceptive effect increases slowly and then is maintained over the assessment period.

Celecoxib alone reached a maximal effect of 63.6 ± 9.9% 2.5 h after its administration, while the celecoxib + methocarbamol 50 mg/Kg combination reached a maximal effect of 78.3 ± 3.3% 2.5 hours after its administration. Four hours after being administered, celecoxib alone maintained an effect of 62.5 ± 7.0%, while celecoxib + methocarbamol 50 mg/g had an effect of 67.8 ± 4.4% (Figure 6).

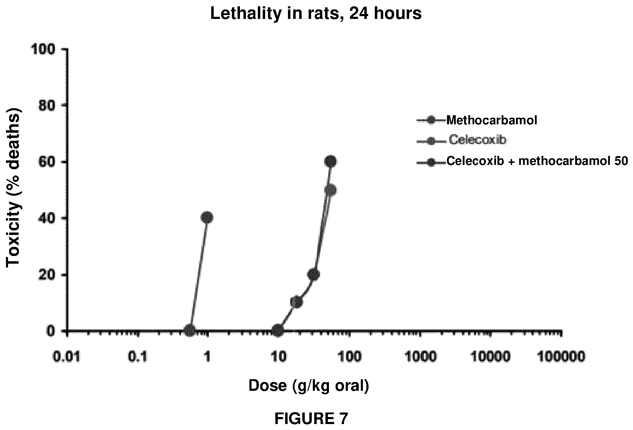

Regarding lethality, Figure 7 shows the DRCs of methocarbamol, of celecoxib, and of the celecoxib + methocarbamol 50 mg/Kg combination in rats 24 hours after being administered. Only part of the lethality DRCs of methocarbamol alone, celecoxib alone and the celecoxib + methocarbamol 50 mg/Kg combination can be presented since a maximal concentration limit was reached.

It can be also noted that the doses needed to trigger lethality effects are much lower than those used in the combination: in the case of methocarbamol, the proposed dose is 50 mg/Kg, much lower than the one causing lethality effects within 24 hours, i.e., 1,000 mg/Kg (1.0 g/Kg). As for celecoxib, the maximal dose for antinociceptive effects in the DRC is 1 g/Kg, while the lethality DRC begins at 17.8 g/Kg, an amount much lower than the dose resulting in the desired effects.

Thus, the doses of celecoxib alone or combined with methocarbamol causing lethality effects within 24 hours are far higher than the suggested therapeutic doses, and there is no evidence that the combination is more toxic than celecoxib alone.

In the current state of the art there are several pharmacological treatments of muscular pain, inflammation, contractions and other derived diseases and symptoms; however, there is no other treatment characterized by the combination of the active agents celecoxib and methocarbamol. The development of this invention provides a real and safe alternative for the control and treatment of pain, and can reduce treatment durations, therapeutic effects and secondary reactions. The separate administration of these compounds requires approximately 0.01 mg to approximately 1000 mg/day for celecoxib, and approximately 0.01 mg to approximately 1000 mg/day for methocarbamol.

This invention was developed to be administered by oral route , either as immediate release of both drugs, or modified release of one or both drugs, with lower doses, higher therapeutic potency and less risk of adverse events.

### EXAMPLES

The following pharmaceutical compositions are described below as nonlimiting examples:

### Example 1 (Invention): Oral administration composition.

| |
|---|
| Celecoxib or pharmaceutically acceptable salt |
| Methocarbamol or pharmaceutically acceptable salt |
| Pharmaceutically acceptable excipient and/or carrier and/or additive |

### Example 2 (Comparative, for illustration purposes only): Intramuscular and intravenous administration composition.

| |
|---|
| Celecoxib or pharmaceutically acceptable salt |
| Methocarbamol or pharmaceutically acceptable salt |
| Pharmaceutically acceptable excipient and/or carrier and/or additive |

### Example 3 (Comparative, for illustration purposes only): Topical administration composition.

| |
|---|
| Celecoxib or pharmaceutically acceptable salt |
| Methocarbamol or pharmaceutically acceptable salt |
| Pharmaceutically acceptable excipient and/or carrier and/or additive |

This invention can be represented in other specific ways without departing from its spirit or essential characteristics, and with any required excipient and/or carrier and/or additive to obtain the desired pharmaceutical form.

The described embodiments will be in any aspect considered as illustrative and not as limitations. Accordingly, the scope of this invention is defined by the appended claims and not by the above description. Its scope encompasses all modifications within the meaning and range of equivalence of the claims.

In an integral way, this invention provides the following advantages:
1. In the acute administration, the celecoxib and methocarbamol 50 mg/Kg combination triggered better antinociceptive effects than celecoxib alone.
2. Chronic treatment with the celecoxib and methocarbamol combination triggered better antinociceptive effects than the acute administration of this combination.
3. In the acute administration, the celecoxib and methocarbamol 50 mg/Kg combination showed better antinociceptive effects in the DRC than celecoxib alone.
4. Chronic treatment with celecoxib triggered more antinociceptive effects than those of the acute administration of celecoxib.
5. Chronic treatment with the celecoxib and methocarbamol combination triggered better antinociceptive effects than the acute administration of this combination.
6. The DRC lethality effects fraction of methocarbamol is more displaced to the left than the DRC of celecoxib, and therefore methocarbamol requires lower dosing ranges to cause the same lethality effect.
7. The lethal effects DRC of the celecoxib and methocarbamol combination remains practically in the same site of the lethal effects DRC of celecoxib alone, whether 24 hours, 48 hours or 72 hours, after treatment. This means that the celecoxib and methocarbamol combination is not more toxic or lethal than celecoxib alone.
8. In order to generate lethal effects with methocarbamol alone, celecoxib alone or with the celecoxib and methocarbamol combination, the doses required are extremely high and far removed from therapeutic doses.

Celecoxib doses used for the chronic treatment DRC, alone or combined, does not modify the normal weight gain of experimental animals, while the high doses used to determine LD₅ do affect weight gain. Weight gain disturbances are similar when administering celecoxib alone or the celecoxib with methocarbamol 50 mg/Kg combination.

## Claims

1. A synergic pharmaceutical combination comprising:
i. a selective cyclooxygenase-2 inhibitor, wherein the selective cyclooxygenase-2 inhibitor is celecoxib or pharmaceutically acceptable salts thereof,
ii. a guaifenesin-derived carbamate agent, wherein the guaifenesin-derived carbamate agent is methocarbamol or pharmaceutically acceptable salts thereof,
iii. a pharmaceutically acceptable carrier and/or excipient and/or additive,
wherein the combination is formulated as a single dosing unit for oral administration,
and wherein the active agent celecoxib is comprised in a concentration of 1000 mg/kg and the active agent methocarbamol is comprised in a concentration of 50 mg/kg;
for use in the treatment of muscular pain, inflammation and contractions in mammals.

2. The pharmaceutical combination for the use according to claim 1, wherein the combination is administered as a single dosing unit in one of the forms selected from the group consisting of: capsules, tablets, lozenges, sublingual tablets, granules, caplets, suspensions, and solutions.

3. The pharmaceutical combination for the use according to any one of claims 1 or 2, wherein the mammal is a human being.

## Patentansprüche

1. Synergistische pharmazeutische Kombination, umfassend:
i. einen selektiven Cyclooxygenase-2-Hemmer, wobei es sich bei dem selektiven Cyclooxygenase-2-Hemmer um Celecoxib oder pharmazeutisch verträgliche Salze davon handelt,
ii. einen von Guaifenesin abgeleiteten Carbamat-Wirkstoff, wobei es sich bei dem von Guaifenesin abgeleiteten Carbamat-Wirkstoff um Methocarbamol oder pharmazeutisch verträgliche Salze davon handelt,
iii. einen pharmazeutisch verträglichen Träger und/oder Hilfsstoff und/oder ein pharmazeutisch verträgliches Additiv,
wobei die Kombination als einzelne Dosiereinheit zur oralen Verabreichung formuliert ist,
und wobei der Wirkstoff Celecoxib in einer Konzentration von 1000 mg/kg vorliegt und der Wirkstoff Methocarbamol in einer Konzentration von 50 mg/kg vorliegt;
zur Verwendung bei der Behandlung von Muskelschmerz, -entzündung und -kontraktion bei Säugern.

2. Pharmazeutische Kombination zur Verwendung nach Anspruch 1, wobei die Kombination als eine einzelne Dosiereinheit in einer der Formen verabreicht wird, die ausgewählt ist aus der Gruppe bestehend aus: Kapseln, Tabletten, Lutschtabletten, Sublingualtabletten, Granulaten, Dragees, Suspensionen und Lösungen.

3. Pharmazeutische Kombination zur Verwendung nach einem der Ansprüche 1 oder 2, wobei der Säuger ein Mensch ist.

## Revendications

1. Combinaison pharmaceutique synergique comprenant :
i. un inhibiteur sélectif de la cyclooxygénase-2, dans laquelle l'inhibiteur sélectif de la cyclooxygénase-2 est le célécoxib ou des sels pharmaceutiquement acceptables de celui-ci,
ii. un agent carbamate dérivé de la guaifénésine, dans laquelle l'agent carbamate dérivé de la guaifénésine est le méthocarbamol ou des sels pharmaceutiquement acceptables de celui-ci,
iii. un support et/ou un excipient et/ou un additif pharmaceutiquement acceptable,
dans laquelle la combinaison est formulée sous la forme d'une unité posologique unique pour administration orale,
et dans laquelle l'agent actif célécoxib est présent à une concentration de 1000 mg/kg et l'agent actif méthocarbamol est présent à une concentration de 50 mg/kg ;
destinée à être utilisée dans le traitement des douleurs musculaires, de l'inflammation et des contractures chez les mammifères.

2. Combinaison pharmaceutique destinée à l'usage selon la revendication 1, dans laquelle la combinaison est administrée sous forme d'une unité posologique unique dans l'une des formes choisies dans le groupe constitué par : des gélules, des comprimés, des pastilles, des comprimés sublinguaux, des granulés, des caplets, des suspensions et des solutions.

3. Combinaison pharmaceutique destinée à l'usage selon l'une quelconque des revendications 1 ou 2, dans laquelle le mammifère est un être humain.
